# EUROPÄISCHE PATENTSCHRIFT

(11) **EP 1 053 258 B1**
(45) Veröffentlichungstag und Bekanntmachung des Hinweises auf die Patenterteilung: **09.04.2003**
(21) Anmeldenummer: 99904837.4
(22) Anmeldetag: 01.02.1999
(51) Int. Cl.: C08F 2/18, C08F 220/56, C08F 220/00, C08F 220/32, C12N 11/08

(54) **VORRICHTUNG ZUR HERSTELLUNG VON TRÄGERPOLYMERMATERIALIEN IN FORM VON PORÖSEN POLYMERPERLEN**
DEVICE FOR PRODUCING POLYMER SUPPORT MATERIALS IN THE FORM OF POROUS POLYMER BEADS
DISPOSITIF POUR LA PRODUCTION DE SUBSTRATS POLYMERES SOUS FORME DE PERLES POLYMERES POREUSES

(30) Priorität: 05.02.1998 DE 19804518
(43) Veröffentlichungstag der Anmeldung: 22.11.2000
(73) Patentinhaber: Röhm GmbH & Co. KG, 64293 Darmstadt (DE)
(72) Erfinder: MEIER, Christian, D-64295 Darmstadt (DE); SÜFKE, Thomas, D-64390 Erzhausen (DE); PETEREIT, Hans-Ulrich, D-64291 Darmstadt (DE); RECKTENWALD, Roger, D-64625 Bensheim (DE)
(86) Internationale Anmeldenummer: EP9900635
(87) Internationale Veröffentlichungsnummer: WO99040122

(56) Entgegenhaltungen:
- US-A- 4 247 643
- US-A- 4 511 694
- US-A- 5 294 491

## Beschreibung

Die Erfindung betrifft ein Verfahren zur Herstellung eines perlförmigen, vernetzten hydrophilen, gegenüber Liganden mit nucleophilen Gruppen bindungsaktiven Mischpolymerisats durch inverse Suspensionspolymerisation einer Monomerenphase. Die Erfindung betrifft weiterhin Trägerpolymermaterialien mit hoher Bindungskapazität für Penicillinamidase und niedriger Quellzahl sowie deren Verwendung

### Stand der Technik

Poröse polymere Trägermaterialien für Proteine, insbesondere Enzyme sind hinreichend bekannt. Anwendungsgebiete liegen im medizinischen Bereich, z. B. bei der enzymatischen Spaltung von β-Lactamantibiotika wie Penicillin G zu 6-Aminopenicillan-Säure (6-APA) mittels der Penicillin-Acylase (Penicillinamidase). Wichtige Entwicklungsziele sind vor allem eine möglichst hohe Beladungskapazität, aber auch eine niedrige Quellbarkeit sowie möglichst geringe Restlösemittelgehalte. Halogenierte Lösungsmittel sollen bei der Herstellung grundsätzlich vermeiden werden.

DE-OS 22 37 316 beschreibt ein Verfahren zur Herstellung perlförmiger, vernetzter Mischpolymerisate durch radikalische Polymerisation eines einen radikalbildenden Initiator enthaltenden Monomergemisches, das ein gegenüber biologischen Substanzen bindungsaktives Monomer, ein vernetzendes Comonomer und wenigstens ein weiteres Comonomer enthält, wobei das Monomerengemisch in einer unpolaren organischen Flüssigkeit zu Tröpfchen suspendiert und polymerisiert wird. Als unpolare organische Flüssigkeit eignen sich insbesondere aliphatische Kohlenwasserstoffe, vor allem solche mit 8 und mehr C-Atomen. In den Beispielen werden Mischungen aus n-Heptan und Perchlorethylen eingesetzt. Das Verhältnis der Monomerenphase zur kontinuierlichen organischen Phase kann zwischen 1 : 1 und 1 : 10 liegen, jedoch werden Verhältnisse zwischen 1 : 1,5 und 1 : 4 bevorzugt. DE-A 31 06 456 beschreibt ein gegenüber DE-OS 22 37 316 in Bezug auf die Bindungskapazität der Polymerperlen verbessertes Verfahren. Besonders hohe Bindungskapazitäten für Proteine, insbesondere für das Enzym Penicillin-Acylase (Penicillinamidase) werden erhalten, wenn die Trägerpolymere hohe Gehalte an vernetzenden Monomeren aufweisen und wenn die Monomerenphase, gebildet aus den Monomeren und dem Verdünnungsmittel, ein Lösungsmittelgemisch als Verdünnungsmittel enthält. Geeignete Gemische können z. B Wasser/Methanol oder Formamid/Methanol sein. Monomere und Verdünnungsmittel liegen etwa im Verhältnis von 1 : 2,6 vor. Für die organische, kontinuierliche Phase wird ein Gemisch n-Hexan und Perchlorethylen verwendet. Das Verhältnis der Monomerenphase zur kontinuierlichen organischen Phase liegt in den Beispielen bei ca. 1 : 2,8. Bei Vernetzeranteilen von 50 Gew.-% im Monomerengemisch und der Verwendung von Wasser/Methanol als Verdünnungsmittel können Trägerpolymere mit einer Bindungskapazität, gemessen als Penicillinacylase-Aktivität, von bis zu 125 U/g erhalten werden.

### Aufgabe und Lösung

Der Erfindung liegt die Aufgabe zugrunde ein verbessertes Verfahren zur Herstellung perlförmiger, vernetzter Mischpolymerisate bereitzustellen. Dabei sollte auf die Verwendung halogenierter Lösungsmittel in der organischen kontinuierlichen Phase verzichtet werden und zugleich eine Bindungskapazität für das Enzym Penicillinamidase (EC 3.5.1.11) unter standardisierten Bedingungen (Beladung von 1g Trägerpolymermaterial mit 1530 Einheiten Penicillinamidase) von mindestens 220 [U/g feucht] erreicht werden. Weiterhin sollte die Quellbarkeit der Polymerperlen in Wasser ausgedrückt in einer Quellungszahl (ml feucht / ml trocken) von nicht mehr als 1,5.

Die Aufgabe wurde gelöst durch ein Verfahren zur Herstellung eines perlförmigen, vernetzten hydrophilen, gegenüber Liganden mit nucleophilen Gruppen bindungsaktiven Mischpolymerisats durch inverse Perlpolymerisation einer Monomerenphase, die aus Monomeren und einem Verdünnungsmittel bestehen, wobei als Monomere
a) 5 - 40 Gew.-% hydrophile radikalisch polymerisierbare Monomere mit einer Vinylgruppe, die bei Raumtemperatur wenigstens 10 %-ige wäßrige Lösungen bilden
b) 30 - 50 Gew.-% radikalisch polymerisierbaren Monomere mit einer Vinylgruppe und einer zusätzlichen funktionellen Gruppe, die in einer polymeranalogen Reaktion mit den nucleophilen Gruppen der Liganden kovalente Bindungen eingehen kann
c) 20 - 60 Gew.-% hydrophile, vernetzende radikalisch polymerisierbare Monomere mit zwei oder mehr ethylenisch ungesättigten polymerisierbaren Gruppen
mit der Maßgabe, daß sich a), b) und c) zu 100 Gew.-% addieren, enthalten sind und als Verdünnungsmittel ein Gemisch aus Methanol und Wasser im Verhältnis 1 : 1,0 bis 1 : 4,0 verwendet wird, wobei die Monomerenphase in einer kontinuierlichen Phase aus einem organischen Lösungsmittel aus einem aliphatischen Kohlenwasserstoff mit 5 - 7 Kohlenstoffatomen zu Tröpfchen verteilt ist, wobei das Verhältnis Monomerenphase zu kontinuierlicher Phase 1 : 2,0 bis 1 : 4,0 beträgt, und in dieser Form in Gegenwart von eines Polymerisationsinitiators und eines Schutzkolloids radikalisch polymerisiert werden, mit der Maßgabe, daß das Verhältnis der Monomeren zum Verdünnungsmittel 1 : 1,7 bis 1 : 2,4 beträgt.

Unter Anwendung des erfindungsgemäßen Verfahrens ist ein neuartiges Trägerpolymermaterial erhältlich, das eine Beladungskapazität für Penicillinamidase von mindestens 220 [U/g feucht], resultierend aus der Umsetzung von 1530 Einheiten Penicillin-Acylase mit 1 g Trägerpolymermaterial, und eine Quelizahl von höchstens 1,5 aufweist.

Es war nicht vorhersehbar, daß die Festlegung der verschiedenen Verfahrensparameter zueinander zu einer deutlich erhöhten Bindungskapazität für das Enzym Penicillinamidase führen würden und gleichzeitig aber die Quellbarkeit abnehmen würde. Überraschend war auch, daß unter Anwendung des erfindungsgemäßen Verfahrens durch die Auswahl des organischen Lösungsmittels aus den aliphatischen Kohlenwasserstoff mit 5 - 7 Kohlenstoffatomen auf den Einsatz halogenierter Kohlenwasserstoffe, wie Perchlorethylen, die bisher vor allem für den Dichteangleich der Phasen verwendet wurden, verzichtet werden kann.

### Ausführung der Erfindung

### Monomere

Um die Hydrophilie des Monomerengemischs zu gewährleisten, muß dieses zum überwiegenden Teil aus hydrophilen Monomeren bestehen. Unter hydrophilen Monomeren sind solche Monomere zu verstehen, die bei Raumtemperatur wenigstens 10 %-ige wäßrige Lösungen bilden und vorzugsweise keine ionischen oder durch Säure- oder Basenzusatz ionisierbaren Gruppen enthalten.

Die Monomere a) sind 5- 40, 8 - 35, insbesondere 9 - 12 Gew.-% hydrophile radikalisch polymerisierbare Monomere mit einer Vinylgruppe, die bei Raumtemperatur wenigstens 10 %-ige wäßrige Lösungen bilden.

Als Monomere a) sind insbesondere Acrylamid und/oder Methacrylamid geeignet, wobei Methacrylamid bevorzugt wird. Weitere Beispiele sind Hydroxyalkylester von ungesättigten polymerisierbaren Carbonsäuren, wie Hydroxyethylacrylat und Hydroxyethylmethacrylat oder N-Vinylpyrrolidon.

Monomere b) sind 30 - 50, bevorzugt 35 - 45 Gew.-% radikalisch polymerisierbaren Monomere mit einer Vinylgruppe und einer zusätzlichen funktionellen Gruppe, bevorzugt einer Oxirangruppe (Epoxygruppe), die in einer polymeranalogen Reaktion mit den nucleophilen Gruppen der Liganden kovalente Bindungen eingehen kann. Insbesondere Oxirangruppen sind geeignet, um Liganden unter Erhaltung ihrer biologischen Aktivität zu binden.

Bevorzugte Monomere b) sind Glycydylmethacrylat und/oder Allylglycidylether. Besonders bevorzugt werden gleichzeitig beide Monomere in etwa gleichen Mengen eingesetzt.

Monomere c) sind 20 - 60, insbesondere 25 - 55, besonders bevorzugt 40 - 55 Gew.-% hydrophile, vernetzende radikalisch polymerisierbare Monomere mit zwei oder mehr ethylenisch ungesättigten polymerisierbaren Gruppen. Bevorzugte Monomere c) sind N,N'-Methylen-bis-Acrylamid oder N,N'-Methylen-bis-Methacrylamid. N,N'-Methylen-bis-Methacrylamid wird besonders bevorzugt. Gegebenenfalls können auch 0 - 10 Gew.-% weiterer vernetzender, radikalisch polymerisierbarer Monomere mit zwei oder mehr ethylenisch ungesättigten polymerisierbaren Gruppen eingesetzt werden. Geeignet sind hydrophile Di(meth)acrylate, wie z. B. Polyethyenoxid-Di(meth)acrylate.

Die Monomere a), b) und c) addieren sich jeweils zu 100 Gew.-%.

### Verdünnungsmittel

Die Monomerenphase besteht aus den Monomeren a) bis c), die in einem Verdünnungsmittel, das ein Gemisch aus Methanol und Wasser im Verhältnis 1 : 1,0 bis 1 : 4,0 sein muß, gelöst sind. Besonders günstige Mischungsverhältnisse für Methanol und Wasser liegen bei 1 : 1,2 bis 1: 2,5, insbesondere bei 1: 1,3 bis 1 : 1,7.

### Verhältnis Monomere zu Verdünnungsmittel

Besonders kritisch ist das Verhältnis Monomere zu Verdünnungsmittel. Dieses muß im Bereich von 1 : 1,7 bis 1 : 2,4, besonders bevorzugt im Bereich von 1,9 bis 2,1 liegen.

### Kontinuierliche Phase

Als kontinuierliche Phase eignet sich ein organisches Lösungsmittel, das ein aliphatischer Kohlenwasserstoff mit 4 bis 7 C-Atomen ist. Bevorzugt ist n-Heptan und besonders bevorzugt Cyclohexan.

### Verhältnis Monomerenphase/kontinuierlicher Phase

Das Verhältnis von der Monomerenphase zur kontinuierlichen Phase, gebildet durch das organische Lösungsmittel muß
bei 1 : 2,0 bis 1 : 4,0, bevorzugt zwischen 1 : 2,8 bis 1 : 3,3.

### Weitere Verfahrensbedingungen

Als weitere Bestandteile enthält die suspendierte Monomerphase in an sich bekannter Weise Polymerisations-Initiatoren, bevorzugt sind schwefelfreie Initiatoren, besonders bevorzugt ist 4,4'-Azobis-(4-Valeriansäure), sowie Schutzkolloide (Emulgatoren), wie z. B. ein Mischpolymerisat aus 95 Teilen n-Butylmethacrylat und 5 Teilen 2-Trimethylammoniumethlymethacrylat-Chlorid mit Molekulargewichten (Gewichtsmittel) im Bereich von 30.000 bis 80.000.

Die Perlpolymerisation (auch als Suspensionspolymerisation bezeichnet) wird ansonsten in bekannter Weise ausgeführt, indem z. B. die kontinuierliche Phase und dem Schutzkolloid vorgelegt wird und die Monomerenphase, in der sich auch der Initiator befindet unter Rühren z. B. bei 40 bis 60 °C in der organischen Phase verteilt wird und anschließend auf 60 - 70 °C erhitzt wird. Das Wasser/Methanol-Gemisch kann z. B. über einen Zeitraum von 6 Stunden nahezu vollständig azeotrop ausgekreist werden. Man läßt den Ansatz für ca. 3 - 5 Stunden zu Ende reagieren und kühlt anschließend auf Raumtemperatur ab. Die entstandenen Perlen werden abgesaugt und z. B. für 12 Stunden im Vakuum getrocknet. Alternativ dazu können die Perlpolymerisate auch abfiltriert und mit Wasser gewaschen werden. Bevorzugt wird ein Trocknung in einem Wirbelschichttrockner vorgenommen, da sich auf diese Weise Lösungsmittelreste besonders effektiv entfernen lassen. Die erhaltenen Polymerperlen (= Trägerpolymermaterial) haben eine Größe im Bereich von 50 bis 500 µm, insbesondere von 120 bis 250 µm. Unter der Bindungskapazität wird diejenige enzymatische Aktivität verstanden, die sich bei maximaler Beladung des Trägerpolymermaterials mit einem bestimmten Enzym erreichen läßt. Ein wichtiges Anwendungsgebiet des erfindungsgemäßen Trägerpolymermaterials ist die Spaltung von Penicillin G zu 6-Aminopenicillan-Säure (6-APA) mittels gebundener Penicillinamidase aus *E. coli.* Die Bindungskapazität wird ausgedrückt als Penicillinamidase-Aktivität in Units pro g Trägerpolymerperlen [U/g feucht]. Die Bindungskapazität der erfindungsgemäßen Trägerpolymerperlen beträgt bei dieser Meßmethode mindestens 220 [U/g feucht].

Die Quellbarkeit der Polymerperlen in Wasser wird ausgedrückt durch die Quellungszahl [ml feucht / ml trocken]. Die erfindungsgemäßen Trägerpolymerperlen weisen eine Quellungszahl von nicht mehr als 1,5 auf.

### Verwendungen der erfindungsgemäßen Trägerpolymermaterialien

Die erfindungsgemäßen Trägerpolymermaterialien können zur kovalenten Bindung von Liganden mittels der vorhandenen Oxirangruppen in Rühr- oder Durchflußreaktoren eingesetzt werden. Dies kann z. B. durch Anlagerung von Proteinen, insbesondere Enzymen, aus konzentrierten Lösungen über kovalente Bindung unter Beibehaltung ihrer biologischen Aktivität erfolgen. Weiterhin können auch Peptide, Aminosäuren, β-Lactamantibiotika, Lipide, Nucleotide, Polynukleotide, niedermolekulare nucleophile Verbindungen oder metalloraganische Verbindungen mit den Oxirangruppen der Trägerperlen umgesetzt werden.

Die mit Liganden beladenen Polymerperlen können in an sich bekannter Weise zur stereospezifischen Synthese von chiralen Substanzen, wie Aminosäuren (d-Phenylalamin, p-Hydroxy-d-phenylalanin, I-tert.-Leucin) oder Arzneimitteln, z. B. von Ibuprofen, eingesetzt werden. Ebenso werden sie als Träger eingesetzt in der enzymatischen Spaltung von Penicillin G zu 6-Aminopenicillinansäure (6-APA), Cephalosporin G zu 7-Aminodesacetoxycephalosporansäure (7-ADCA) oder Cephalosporin C zu 7-Aminocephalosporansäure (7-ACA). Das Verfahren ist beschrieben in DECHEMA Jahrestagung 1996 - Kurzfassungen, Bd. 1, DECHEMA e.V.. Weitere Anwendungsgebiete sind spezifische enzymatische Synthesen an Substraten wie z. B. obigen Spaltprodukten zu Amoxicillin und Ampicillin. Ein weiteres Anwendungsgebiet sind Synthesen von Feinchemikalien oder Grundprodukten für chemische Synthesen (z. B. Apfelsäure). Die Polymerperlen können auch in der Separationstechnik zur Adsorptionchromatographie oder Gelpermeationschromatographie verwendet werden. Zur spezifischen Adsorption können die Polymerperlen mit Immunglobulin-Fraktionen aus Antiseren oder mit monoklonalen Antikörper beladen werden. Als weiteres Einsatzgebiet ist die Verwendung des mit Enzymen oder Antikörpern beladenen Trägerpolymermaterials als Adsorbens in der extrakorporalen Therapie, in der pathogene bzw. toxische Substanzen aus Vollblut entfernt werden, zu nennen.

### Beispiele

(Die folgende Bestimmungsmethode ist dem Fachmann auf den Gebiet poröser Trägerpolymermaterialien an sich geläufig und wird nur der Vollständigkeit halber aufgeführt)

### Bestimmung der Bindungskapazität für Penicillinamidase (= Penicillin G-Acvlase) aus E. coli (EC 3.5.1.11)

### a) Kovalente Bindung von Penicillinamidase an das Trägerpolymermaterial

1 g Trägerpolymermaterial werden zu 1530 Units Penicillinamidase in 5 ml sterilem 1 M Kalium-Phosphat-Puffer pH 7,5 gegeben und für 48 h bei 23 °C inkubiert.

Anschließend werden die Polymerperlen auf eine Fritte aus gesintertem Glas (Porösität 2 oder 3) gegeben und zweimal mit entionisierten Wasser und anschließend zweimal mit 0,1 M Kalium-Phosphatpuffer pH 7,5, enthaltend 0,05 % Ethyl-4-hydroxybenzoat, mittels Absaugens auf der Fritte gewaschen. Das Feuchtgewicht der erhaltenen, mit Penicillin-Acylase beladenen Perlen wird bestimmt.

### b) Bestimmung der Bindungskapazität

250 - 300 mg feuchtes mit Penicillinamidase gekoppeltes Trägerpolymermaterial (Polymerperlen) werden in 20 ml einer 2 %-igen Penicillin-G-Lösung in 0,05 M Kalium-Phosphatpuffer pH 7,5, enthaltend 0,05 % Ethyl-4-hydroxybenzoat, bei 37 °C gegeben. Unter gleichmäßiger Rührung erfolgt eine Titration frei gewordener Phenylessigsäure mit 0,5 M NaOH bei einem konstantem pH-Wert von 7.8 für die Dauer von 10 Minuten, wobei der Verbrauch an NaOH aufgezeichnet wird.

Anschließend werden die Polymerperlen wie unter a) über eine Glasfritte mittels Durchsaugen von 20 ml 0,05 M Kalium-Phosphatpuffer pH 7,5, enthaltend 0,05 % Ethyl-4-hydroxybenzoat, gewonnen und die Messung zweimal wiederholt.

### c) Berechnung der Bindungskapazität

Der lienare Bereich der Meßkurven (üblicherweise der Bereich von 1 - 5 min) wird für die Berechnung zugrunde gelegt und auf ein 10 min Interval extrapoliert. Die Bindungskapazität wird als Penicillinamidase Einheiten pro g feuchten Trägerpolymermaterials (U/g feucht) angegeben. Eine Einheit entspricht einem µmol hydrolysiertem Penicillin G pro Minute (µmol/min); 1 I 0,5M NaOH ist dabei äquivalent zu 500 µmol hydrolysiertem Penicillin G. (Der Wassergehalt des Trägerpolymermaterials ist in etwa konstant und kann daher vernachlässigt werden.)

### Beipiele 1 - 3

### Übereinstimmende Versuchsbedingungen in den Beipiele 1 - 3:

In einem 2 I Rührkolben mit Thermometer, Wasserabscheider, Rückflußkühler, Stickstoffeinleitungsrohr werden ein organisches Lösungsmittel, 3 g eines Mischpolymerisats aus 95-Teilen n-Butylmethacrylat und 5 Teilen 2-Trimethylammoniumethlymethacrylat-Chlorid als Schutzkolloid und 5 g Trockeneis vorgelegt. Unter Rühren und Durchleiten von Stickstoff wird bei 50 °C eine Monomerenphase bestehend aus Wasser und Methanol und im Verhältnis 1 : 1,5 als Verdünnungsmittel, sowie
10 g Methacyrlamid,
20 g Allylglycidylether,
20 g Glycidylmethacrylat und
50 g Methylen-bis-methacrylamid
   sowie
2 g 4,4'-Azobis-4-cyanovaleriansäure (als Polymerisationsinitiator)
in der organischen Phase verteilt und anschließend zum Sieden bei 65 - 70 °C erhitzt. Der Ansatz wird für ca. 6 h inkubiert und anschließend auf Raumtemperatur abgekühlt. Die entstandenen Polymerperlen werden abgesaugt, gewaschen und im Wirbelschichttrockner getrocknet. Anschließend wird die Bindungskapazität für Penicillinamidase [U/g feucht] und die Quellzahl bestimmt [ml feucht/ml trocken] bestimmt.

Die wesentlichen Versuchsparameter und die Ergebnisse der Beispiele 1 - 3 sind der nachstehenden Tabelle zu entnehmen.

| | Beispiel 1 (erfindungsgemäß) | Beispiel 2 (Vergleichsbeispiel) | Beispiel 3 (Vergleichsbeispiel) |
|---|---|---|---|
| Organisches Lösungsmittel (kontinuierliche Phase) | 952 g Cyclohexan | 669 g Cyclohexan | 530 g n-Heptan + 530 g Perchlorethylen |
| Monomere insgesamt | 100 g | 100 g | 100 g |
| Verdünnungsmittel | 80 g Methanol + 120 g Wasser (= 1 : 1,5) | 263 g Formamid | 264 g Formamid |
| Monomere + Verdünnungsmittel (Monomerenphase) | 300 g | 363 g | 364 g |
| Verhältnis Monomere/ Verdünnungsmittel | 1 : 2 | 1 : 2,63 | 1 : 2,64 |
| Verhältnis Monomerenphase/ kontinuierliche Phase | 1 : 3,2 | 1 : 1,8 | 1 : 2,9 |
| Bindungskapazität für Penicillinamidase (1530 U) [U/g feucht] | 252 | 194 | 192 |
| Quellzahl [ml feucht/ ml trocken] | 1.3 | 4,0 | 3,9 |

## Patentansprüche

1. Verfahren zur Herstellung eines perlförmigen, vernetzten hydrophilen, gegenüber Liganden mit nucleophilen Gruppen bindungsaktiven Mischpolymerisats durch inverse Perlpolymerisation einer Monomerenphase, die aus Monomeren und einem Verdünnungsmittel bestehen, wobei als Monomere
a) 5 - 40 Gew.-% hydrophile radikalisch polymerisierbare Monomere mit einer Vinylgruppe, die bei Raumtemperatur wenigstens 10 %-ige wäßrige Lösungen bilden
b) 30 - 50 Gew.-% radikalisch polymerisierbaren Monomere mit einer Vinylgruppe und einer zusätzlichen funktionellen Gruppe, die in einer polymeranalogen Reaktion mit den nucleophilen Gruppen der Liganden kovalente Bindungen eingehen kann
c) 20 - 60 Gew.-% vernetzende radikalisch polymerisierbare Monomere mit zwei oder mehr ethylenisch ungesättigten polymerisierbaren Gruppen
mit der Maßgabe, daß sich a), b) und c) zu 100 Gew.-% addieren, enthalten sind und als Verdünnungsmittel ein Gemisch aus Methanol und Wasser im Verhältnis 1 : 1,0 bis 1 : 4,0 verwendet wird, wobei die Monomerenphase in einer kontinuierlichen Phase aus einem organischen Lösungsmittel aus einem aliphatischen Kohlenwasserstoff mit 5 - 7 Kohlenstoffatomen zu Tröpfchen verteilt ist, wobei das Verhältnis Monomerenphase zu kontinuierlicher Phase 1 : 2,0 bis 1 : 4,0 beträgt, und in dieser Form in Gegenwart von eines Polymerisationsinitiators und eines Schutzkolloids radikalisch polymerisiert werden, mit der Maßgabe, daß das Verhältnis der Monomeren zum Verdünnungsmittel 1 : 1,7 bis 1 : 2,4 beträgt.

2. Verfahren nach Anspruch 1, **dadurch gekennzeichnet, daß** als Monomere
a) Acrylamid und/oder Methacrylamid
b) Glycidylmethacrylat und/oder Allylglycidylether
c) Methylen-bis-Acrylamid oder Methylen-bis-Methacrylamid
eingesetzt werden.

3. Verfahren nach Anspruch 1, **dadurch gekennzeichnet, daß** als organisches Lösungsmittel Cyclohexan verwendet wird.

4. Trägerpolymermaterial herstellbar nach einem Verfahren nach einem oder mehreren der Ansprüche 1 bis 3, **dadurch gekennzeichnet, daß** es eine Bindungskapazität für Penicillinamidase aus *E. coli* von mindestens 220 [U/g feucht], resultiernd aus der Umsetzung von 1530 Einheiten Penicillinamidase mit 1 g Trägerpolymermaterial, und eine Quellzahl von höchstens 1,5 aufweist.

5. Verwendung des Trägerpolymermaterials gemäß Anspruch 4 zur Bindung von Proteinen.

6. Verwendung des Trägerpolymermaterials gemäß Anspruch 5 zur Bindung von Enzymen

7. Verwendung des Trägerpolymermaterials gemäß Anspruch 5 zur Bindung von Antikörpern

8. Verwendung des Trägerpolymermaterials gemäß Anspruch 4 in der Chromatographie

9. Verwendung des Trägerpolymermaterials gemäß Anspruch 4 zur Synthese von Arzneistoffen

10. Verwendung des Trägerpolymermaterials gemäß Anspruch 4 zur stereospezifischen Synthese von chiralen Substanzen.

## Claims

1. Process for producing a cross-linked hydrophilic copolymer in bead form which actively binds to ligands with nucleophilic groups, by inverse bead polymerisation of a monomer phase, consisting of monomers and a diluent, wherein the monomers present comprise:
a) 5 to 40 wt.% of hydrophilic, radically polymerisable monomers with a vinyl group which form at least 10% aqueous solutions at ambient temperature
b) 30-50 wt.% of radically polymerisable monomers with a vinyl group and an additional functional group which can form covalent bonds in a polymer-analogous reaction with the nucleophilic groups of the ligands
c) 20 - 60 wt.% of cross-linking, radically polymerisable monomers with two or more ethylenically unsaturated polymerisable groups
with the proviso that a), b) and c) add up to 100 wt.%, and as diluent a mixture of methanol and water in the ratio 1 : 1.0 to 1 : 4.0 is used, the monomer phase being distributed in droplet form in a continuous phase consisting of an organic solvent comprising an aliphatic hydrocarbon having 5 to 7 carbon atoms, the ratio of monomer phase to continuous phase being 1 : 2.0 to 1 : 4.0, and is radically polymerised in this form in the presence of a polymerisation initiator and a protective colloid, with the proviso that the ratio of the monomers to the diluent is 1 : 1.7 to 1 : 2.4.

2. Process according to claim 1, **characterised in that**
a) acrylamide and/or methacrylamide
b) glycidyl methacrylate and/or allylglycidyl ether
c) methylene-bis-acrylamide or methylene-bis-methacrylamide
are used as monomers.

3. Process according to claim 1, **characterised in that** cyclohexane is used as the organic solvent.

4. Carrier polymer material which may be prepared by a process according to one or more of claims 1 to 3, **characterised in that** it has a binding capacity for penicillinamidase from *E. coli* of at least 220 [U/g wet], resulting from the reaction of 1530 units of penicillinamidase with 1 g of carrier polymer material, and a swelling number of at most 1.5.

5. Use of the carrier polymer material according to claim 4 for binding proteins.

6. Use of the carrier polymer material according to claim 5 for binding enzymes.

7. Use of the carrier polymer material according to claim 5 for binding antibodies.

8. Use of the carrier polymer material according to claim 4 in chromatography.

9. Use of the carrier polymer material according to claim 4 for the synthesis of pharmaceutical products.

10. Use of the carrier polymer material according to claim 4 for the stereospecific synthesis of chiral substances.

## Revendications

1. Procédé de production d'un polymérisat mixte sous forme de perles, hydrophile réticulé, actif pour la liaison vis-à-vis de ligands ayant des groupes nucléophiles, par polymérisation inverse en perles d'une phase de monomères, qui consistent en des monomères et en un agent de dilution, dans lequel sont contenus comme monomères :
a) de 5 à 40 % en poids de monomères hydrophiles polymérisables par voie radicalaire ayant un groupe vinyle, et qui forment à température ambiante des solutions aqueuses d'au moins à 10 % ;
b) de 30 à 50 % en poids de monomères polymérisables par voie radicalaire ayant un groupe vinyle et un groupe fonctionnel additionnel qui peut intervenir dans une réaction analogue à la polymérisation avec les groupes nucléophiles, des ligands, des liaisons covalentes,
c) de 20 à 60 % en poids de monomères polymérisables par voie radicalaire, réticulants, ayant deux ou davantage de groupes polymérisables non saturés éthyliniquement,
avec la restriction que a), b) et c) s'additionnent à 100 % en poids,
et comme agent diluant on utilise un mélange à base de méthanol et d'eau dans la proportion de 1 : 1,0 à 1 : 4,0,
dans lequel la phase de monomères est répartie en gouttelettes en une phase continue à base d'un solvant organique consistant en un hydrocarbure aliphatique ayant de 5 à 7 atomes de carbone, le rapport phase de monomères à phase continue étant de 1 : 2,0 à 1 : 4,0, et on polymérise sous cette forme les monomères par voie radicalaire en présence d'un initiateur de polymérisation et d'un colloïde protecteur, avec la restriction que le rapport des monomères à l'agent diluant est de 1 :1,7 à 1 : 2,4.

2. Procédé selon la revendication 1,
**caractérisé en ce que**
comme monomères on met en oeuvre
a) de l'acrylamide et/ou du méthacrylamide ;
b) du méthacrylate de glycidyle et/ou un éther d'allyle et de glycidyle ;
c) du méthylène-bis-acrylamide ou du méthylène-bis-méthacrylamide.

3. Procédé selon la revendication 1,
**caractérisé en ce que**
comme solvant organique on utilise du cyclohexane.

4. Matériau polymère de support qu'on peut produire selon un procédé selon une ou plusieurs des revendications 1 à 3,
**caractérisé en ce que**
il possède une capacité de liaison pour la penicillinamidase provenant d'E. Coli d'au moins 220 U/g humide résultant de la réaction de 1530 unités de penicillinamidase avec 1 g de matériau polymère de support, et un indice de gonflement d'au maximum 1,5.

5. Utilisation du matériau polymère de support conformément à la revendication 4 pour la liaison de protéines.

6. Utilisation du matériau polymère de support conformément à la revendication 5, pour la liaison d'enzymes.

7. Utilisation du matériau polymère de support conformément à la revendication 5 en vue de la liaison des anticorps.

8. Utilisation du matériau polymère de support conformément à la revendication 4, en chromatographie.

9. Utilisation du matériau polymère de support conformément à la revendication 4, en vue de la synthèse de médicaments.

10. Utilisation du matériau polymère de support conformément à la revendication 4, en vue de la synthèse stéréo spécifique de substances chirales.
